# EUROPEAN PATENT SPECIFICATION

(11) **EP 3 677 166 B1**
(45) Date of publication and mention of the grant of the patent: **31.08.2022**
(21) Application number: 19150574.2
(22) Date of filing: 07.01.2019
(51) Int. Cl.: A61B 1/00, G01M 11/02, G01M 11/00

(54) **TEST APPARATUS AND TEST SYSTEM FOR AN OPTICAL SYSTEM, OPTICAL SYSTEM COMPRISING A TEST SYSTEM AND METHOD FOR TESTING AN OPTICAL SYSTEM**
TESTVORRICHTUNG UND TESTSYSTEM FÜR EIN OPTISCHES SYSTEM, OPTISCHES SYSTEM MIT EINEM TESTSYSTEM UND VERFAHREN ZUM TESTEN EINES OPTISCHEN SYSTEMS
APPAREIL D'ESSAI ET SYSTÈME D'ESSAI POUR SYSTÈME OPTIQUE, SYSTÈME OPTIQUE COMPRENANT UN SYSTÈME D'ESSAI ET PROCÉDÉ D'ESSAI D'UN SYSTÈME OPTIQUE

(43) Date of publication of application: 08.07.2020
(73) Proprietor: Maxer Endoscopy GmbH, 78573 Wurmlingen (DE)
(72) Inventor: JOSHI, Shirish, 78573 Wurmlingen (DE)
(74) Representative: Patentanwälte und Rechtsanwalt Weiß, Arat & Partner mbB

(56) References cited:
- EP-A1- 2 526 851
- EP-A2- 2 449 959
- US-A- 5 115 126
- US-A1- 2003 107 726
- US-A1- 2008 023 625
- US-A1- 2011 140 003
- US-A1- 2014 246 563
- US-A1- 2014 267 656

## Description

### Technical Area

The present disclosure is directed at method for testing an optical system, a test system and an optical system comprising such a test system.

### State of the Art

Methods, systems and apparatuses for testing optical systems are widely known.

Those optical systems may be endoscopes or other optical systems used in the filed of medicine. Also, other optical systems for use outside the field of medicine are known, for example borescopes.

Nowadays, video endoscopy systems are widely used. The image provided by such a system depends, among others, on the luminous flux provided by the system to illuminate the area under observation. Currently, hospitals do not have an easy way to measure that light output, which leads, among others, to two major problems: First, the user such as a surgeon has to accept inferior results during surgery. Second, since not even the light output can be measured by the surgeon, the cause of a bad image cannot easily be found. Therefore, even if the problem could easily be fixed by the people in the operating room, often the whole endoscopic system is send to maintenance department consuming costs and time in often critical situations.

Devices are known for testing optical systems such as endoscopes.

Prior art document US2014/246563 discloses an endoscopic system according to the preamble of claim 1 and also discloses a method for testing such a system.

For example, US 2011 0 140 003 A1 describes a test apparatus for an optical investigation system such as an endoscope. The test apparatus comprises a reference body which in turn comprises an indicator area and a reference area. A mechanical stop is provided ensuring the endoscope to be tested to be placed as requested in order to achieve a good measurement result. Since the test apparatus disclosed by US 2011 0 140 003 A1 does not comprise a light sensor it is not possible to test individual components of the endoscope separately.

US application US 5 115 126 A also discloses a means for checking endoscope equipment, i.e. the light-guide cable and the lamp. A beam splitter and two separate detection means are provided in order to check both the lamp and the light-guide cable automatically. The apparatus of US 5 115 126 A is very complex and needs high-maintenance.

US application US 2003 0 107 726 A1 criticizes devices as the one describe in US 5 115 126 A above because they rely on two detections means or sensors which need to be calibrated in order to draw usable conclusions. Moreover, US 2003 0 107 726 A1 criticizes that the device according to US 5 115 126 A does not include its own light source thus relying on the light source of the endoscopic system to be tested. To overcome these drawbacks, US 2003 0 107 726 A1 discloses a system comprising a spherical chamber with an opening to accommodate an outlet portion of an endoscope. The chamber further comprises a light sensor. An external light source is provided so that the system can be operated independent of the light source of the endoscopic system. The apparatus described in US 2003 0 107 726 A1 only allows for detection of transmission losses originating in the light guide means, whereas defects of the light source cannot be tested.

### Problem to be solved

The present invention seeks to overcome the drawbacks of prior art systems. The invention and preferred embodiments thereof is defined solely in claims 1 - 5. All unclaimed examples or configurations disclosed in the description are for exemplary purposes only.

### Solution of the Problem

**A cap** for covering a radiation exit region of a part of an optical system to be tested by measuring a luminous flux comprises, according to the present invention, a holding means and a lid portion. Preferably, the cap is sterilisable. A radiation exit region is the region or portion or segment where the radiation leaves the part of the optical system to be tested. In common endoscopes, the outlet portion at the distal end which is during surgery inserted in the body of the patient is such a radiation exit region.

The part to be tested may, for example, be any part which generates radiation or where radiation is passed through or amplified or damped. For example, such a part may be an endoscope of an endoscopic system or a light guide means such as light guide cable.

According to the present disclosure, an optical system preferably comprises at least one optical instrument such as an endoscope. According to the present disclosure, a single optical instrument already constitutes an optical system. However, usually optical systems comprise an optical instrument and other parts and/or devices.

The holding means may be any means capable of holding, fixing or attaching the lid portion to that area where the radiation leaves the radiation exit region. The holding means may be a magnetic holder or a clip.

The holding means may be permanently fixed to the lid portion. Alternatively, the holding means may be detachable from the lid portion.

The holding means may also be a simple marking on the cap enabling the user to hold the cap in place. In this case the endoscope described hereafter may comprise a corresponding marking so that the user immediately knows how to place both marking next or on top of each other to guarantee correct placement of the cap.

Alternatively, the holding means may be a wall portion. Preferably, the wall portion is cylindrically shaped and the lid portion provides a closure on one side of the wall. However, other shapes are encompassed by the present invention; both portions may also be blended or merged into each other without a definite border between lid portion and wall portion. Preferably, however, the wall portion embraces the shell or housing of the radiation exit region and the lid portion covers the area where the radiation leaves the radiation exit region. The cap is sterilisable. The cap may be made from a thermostable material such as a thermostable plastics material which can be sterilized by steam sterilization. The cap may also be made from a material resistant to chemical agents used for chemical sterilization so that it can be chemically sterilized using appropriate chemical agents.

The cap may be sterilisable one time or multiple times. Furthermore, the cap may be user sterilisable.

Using a sterile cap is advantageous because the test employing the cap which is described in detail hereafter may be conducted during the surgery without the need of sterilizing the optical instrument such as an endoscope after conducting the test. In case the radiation exit region which is usually an end portion of the optical instrument to be tested gets in contact with an item which is not sterile the cap prevents the radiation exit region from becoming nonsterile.

As described hereinafter, the test to be conducted is preferably a test for assessing luminous flux.

At least the wall portion may be flexible, for example, it may be made from a flexible material. Thus, size deviations of the part to be tested, for example deviations of the radius of the radiation exit region, can be easily compensated for. Also, a flexible cap may sit tightly on the radiation exit region in case it is stretched when putting it in place, preventing undesired falling off.

The cap may be a single-use-item or it may be reusable, for example, by cleaning and sterilizing it. A single-use-item is easy to use and mistakes during sterilization by the user are avoided, while the a reusable cap is advantageous for the environment since it is resource saving.

At least the lid portion may have a transmission degree for radiation of a pre-defined wavelength range between 0.0001% and 50%. The transmission degree of a material is a quotient which is obtained by dividing the intensity before the radiation passes the material by the intensity after the radiation passed the material. Since the transmission degree depends on the thickness of the material, on the angle of incidence and on the wavelength of the radiation, herein the transmission degree refers to an individual item which is placed substantially orthogonal to a bundle of rays.

The inverse value of the transmission degree is opacity. Thus, in other words, the lid portion may be or comprise a opacity-reducing device.

The transmission degree for a pre-defined wavelength range may also be between 0.0001% and one of the following values: 25%, 15%, 10%, 5%, 1%, 0.1% or 0.01%.

The transmission degree for a pre-defined wavelength range may also be between 0.001% and one of the following values: 25%, 15%, 10%, 5%, 1%, 0.1% or 0.01%.

The transmission degree for a pre-defined wavelength range may also be between 0.01% and one of the following values: 25%, 15%, 10%, 5%, 1%, and 0.1%.

The transmission degree for a pre-defined wavelength range may also be between 0.1% and one of the following values: 25%, 15%, 10%, 5% or 1%.

The transmission degree for a pre-defined wavelength range may also be between 0.5% and one of the following values: 25%, 15%, 10%, 5% or 1%.

The transmission degree for a pre-defined wavelength range may also be between 5% and one of the following values: 25%, 15% or 10%.

The transmission degree mentioned above may be provided by different mechanisms, for example, by absorption or by scattering or by a combination of such mechanisms.

The lid portion may either be made from a material providing such a transmission degree or it may comprise an optical device, for example, such as a lens or a diffusor which provides such a transmission degree.

A cap provided with a transmission degree as stated above is advantageous because it can be used with basic prior art radiation sensors which would be saturated without the transmission reduction, which would then render a quantification of the luminous flux, for example, impossible. By using the cap no adjustments have to be made with regard to the light source or the sensor used in order to conduct a comprehensive test including a quantitative or at least qualitative statement concerning luminous flux or the like.

When referring to a sensor hereafter, usually the radiation sensor is meant.

As mentioned above, the cap may comprise or act as a lens or a diffusor. Besides providing the desired transmission degree, i.e. being opacity-reducing, such embodiments provide other advantages.

When the lid portion acts as or comprises a diffusor, a ring-shaped bundle of rays may be homogenized so that instead of a ring-shaped bundle of rays a substantially homogenous beam leaves the cap. Such ring-shaped bundles of rays often occur in common endoscopes because in the middle of that ring the image sensor or the light guide means which transport the image to the image sensor are located.

By comprising or acting as a diffusor or a similar device, the lid portion may be configured to scatter the radiation of a pre-defined wavelength range, thus providing a beam with substantially generated homogenous illumination.

The lid portion may absorb radiation, for example, by comprising or by being made of semi-transparent material.

The lid portion may act as or comprise a lens, which may align or level the bundle of rays as desired by the user.

The lid portion may, according to an alternative embodiment, also be located somewhere in the middle of the wall portion and not at its end. Thus, a pre-defined distance between the lid portion and the radiation sensor is complied with, when the cap is hold against the sensor.

The cap may also comprise a hold point such as an edge or a protuberance in order to ensure that between the very distal end of the endoscope, for example, and the lid portion a pre-defined distance is complied with.

A cap as described above guarantees repeatable measurement results.

The cap may provide further advantages. Oftentimes the distal end of an endoscope has to be pre-heated prior to the surgery because when the cold endoscope is introduced into the patient's body and the radiation source is switched on thereafter, fogging cannot be avoided which limits the vision of the surgeon.

The cap according to the present disclosure may therefore be configured to absorb radiation and in turn heat up, which will also lead to a heating-up of the distal end of the endoscope. Using a cap for pre-heating is described with respect to infrared radiation in DE 43 16 623 C2. The current application also encompasses pre-heating by using materials which absorb visible light radiation and thus do not rely on infrared radiation for pre-heating.

The cap may further comprise a temperature indication means. Such a means may indicate the temperature of the cap and consequently also the temperature of the part of the endoscope covered by the cap. For example, colour sensitive ink which changes its colour in response to temperature variations may be employed. The cap or a part of the cap may also be made from a material which responds to temperature changes by changing its colour.

The cap may comprise a means for preventing overheating. For example, a temperature-sensitive predetermined breaking point may be used. When the temperature rises above a certain pre-defined level, the means breaks allowing the radiation to spread into a bigger area without being absorbed by the cap thus preventing overheating. Moreover, after that rupture the light will be more easily visible from outside so the user immediately registers overheating.

Since the transmission degree depends on the wavelengths, with respect to visible light on its colour, different caps may be used for radiation of different wavelengths. Using a cap which is optimized for a certain wavelength the desired reduction of transmission can be reached. However, it is also possible to use the same cap for radiation of various wavelengths when the transmission for all those wavelengths is reduced below a certain level.

The inner surface of the cap may be reflective, similar to a light guide means, in order to ensure the radiation to be transported to the lid portion and to exit the cap.

The size and shape of the cap may be fitted to the sensor it is to be used with. An end portion of the cap where the radiation leaves the cap may for example be broadened or tapered.

**The present** disclosure **also comprises a test apparatus for an optical system.** The optical system comprises at least a part to be tested. The test apparatus comprises a radiation sensor which is configured so that the part to be tested can be directly resting on the radiation sensor.

The optical system to be tested may be a system used in the field of medicine such as an endoscopic system, for example.

The type of radiation sensor is advantageously chosen with regard to the wavelength under observation. For example, a photosensor may be chosen. According to the present invention, visible light, infrared radiation or ultraviolet radiation may be observed. Sensors may be chosen accordingly and may be suitable for detecting either one of the aforementioned radiation ranges or subranges of those ranges or even more than one of those ranges. For example, sensors capable of detecting only near infrared radiation may be used in a certain context, while in another situation it would be more appropriate to use a sensor capable of detecting a broad range of, for example, the whole visible spectrum and infrared radiation.

In the context of the present disclosure, directly resting on the sensor also comprises examples where the sensor comprises, for example, a transparent protective layer or the like and the part to be tested, thus, directly rests on that protective layer.

Preferably, of course, the radiation exit region rests on the sensor in a way so that the radiation leaving the part to be tested is directed at and can be registered by the sensor.

The test apparatus may comprise its own processing unit and screen. However, the test apparatus may also be connectable to the optical system to be tested, wherein the optical system usually comprises parts such as a processing unit as part of a control unit, a screen and other parts. Thus, those parts do not have to be included in the test apparatus but are instead shared between the test apparatus and the optical system.

Within the scope of the present disclosure, a test apparatus usually refers to an apparatus such as the one described above, while a test system as described hereinafter comprises not only the test apparatus but also a device for transmission reduction such as a cap as described in detail above.

The present invention comprises an endoscopic system comprising an endoscope and a test system.

The test system comprises a radiation sensor and a device for transmission reduction which is configured to be placed between the part to be tested and the radiation sensor.

The test system is for testing endoscopes comprising a light guide means.

The device for transmission reduction is a cap as described in detail above. However, other devices for transmission reduction are also disclosed. For example, instead of the radiation exit region of the part to be tested also the sensor may be covered by that device. For example, the sensor may be equipped with a lid or disc which may reversibly be positioned to cover the sensor surface.

The test system described above combines the advantages described with respect to the cap and with respect to the test apparatus. When using a lid, a disc or the like instead of a cap, the transmission-reducing effect is the same and the lid, disc or the like is as easy to handle as the cap.

The test system comprises a radiation sensor and a device for transmission reduction which is configured to be placed between the part to be tested and the radiation sensor. The device for transmission reduction is a cap as described in detail above or a lid or a disc as described above.

The optical system and the test system may be separate parts. Preferably, however, the optical system comprises a control unit and an optical instrument, wherein the test system is a built-in part of the control unit. According to Z an example the test system comprises a radiation sensor which is a built-in part of the control unit of the optical system. The radiation sensor may be inserted in the housing of the control unit. In general, the radiation sensor may be inserted or attached at any place which is easily accessible for the user. The radiation sensor may, for example, be inserted or attached to a camera, to the flat panel display, to the light source or its housing or to an insufflator.

Thus, the radiation sensor provides for an integrated luxmeter.

Control units for optical systems such as endoscopic systems are known. They may comprise all the processing means, control means, switches, interfaces and ports required for operating the system. Thus, the user such as a surgeon only holds the optical instrument such as an endoscope in his hand, while other interconnected parts which are outsourced to the control unit. If the radiation source is integrated into the control unit, there may be a light guide means connecting the control unit and the optical instrument. Those arrangements are known.

Other examples are also contemplated. For example both, the optical system and/or the test system may be composed of multiple parts, wherein at least one part may be a part which is shared by both systems. All the parts may be interconnected by commonly known means such as cables and/or wireless connections.

The optical system described above is, thus, advantageously equipped with an easy-to-handle test system as described in detail herein.

The present invention also comprises a **method for testing an** endoscopic system comprising an endoscope and a test system according to claim 2 with claims 3 - 5 defining preferred embodiments. The method comprises the following steps:
- Holding a radiation exit region of the part to be tested directly against a radiation sensor
- Activating a radiation source providing the part to be tested with radiation
- Providing a feedback on luminous flux registered by the radiation sensor

The aforementioned steps may be conducted in the given order. Alternatively, the radiation source may be activated earlier as described above.

The optical system may comprise the test system or at least the radiation sensor as described above.

The method is suitably used in the medical field, for example when testing endoscopic systems. However, the method may also be employed outside the medical field.

The optical system which is to be tested may also consist only of an optical instrument such as an endoscope. However, usually optical systems such as endoscopic systems are used nowadays wherein the hand-held endoscope is connected to a control unit which comprises a data processing device and a radiation source. Thus, optical systems often comprise more than one part.

Preferably, the radiation exit region is located or placed relative to the sensor so that all the radiation leaving the part arrives at the sensor.

The feedback can be, for example, a visual feedback or an acoustic feedback. The visual feedback may be given on the flat panel display, e.g. by appropriate pictograms, numbers or the like.

The feedback may be generated by comparing the luminous flux registered by the radiation sensor with luminous flux values stored in the system or with luminous flux registered during former surgeries or with luminous flux values pre-defined by the user or a combination of the aforementioned values. Suitably, at least a first lower border (minimum) of luminous flux range is stored in the system in order detect deterioration. There may be a second lower border, wherein the first lower border indicates beginning deterioration of the radiation which still allows the system to be used while the second lower border indicates that the system should not be used any more.

After comparing the values the system may for example indicate the deviation of the luminous flux from the values stored in the system. This may be done by giving the deviation in percent or by providing the luminous flux together with the registered information whether that luminous flux is above at least one of the lower borders. In short, for example relative or absolute luminous flux values may be given which may also be translated into qualitative statements which can readily be interpreted by the user.

Additionally or alternatively the feedback may include instructions and guidance to the user. For example, the user may be asked to conduct a sequence of steps such as the three steps described below. While these steps may, according to the embodiment described below, be conducted under the direction of the user or surgeon, it is also possible that those steps are headed by the system and the method described above in giving directions to the user. For example, the method or system may ask the user first to hold the radiation exit end of the endoscope against the radiation sensor. Thereafter, the luminous flux is registered and the user may be asked to confirm that step by pressing a button. In this way of requests to perform a certain action followed by requests to confirm, the user may be guided through the steps described hereafter. By guiding the user as described above, errors are avoided and distraction of the user is minimized.

The method described above provides for an easy and reliable way of testing and error tracking. For example, if the endoscopic system to be used in surgery does not provide the desired image quality, the surgeon may just conduct the method by first holding the radiation exit region of the endoscope, usually the distal end of the endoscope, against the radiation sensor. After that first step the surgeon receives the feedback and knows whether the hardware of the endoscopic system such as the radiation source, the light guide means or the endoscope itself causes the problem or whether the problem is caused by a software or results from something else such as the image sensor of the endoscope.

In case the hardware of the endoscopic system causes the problem, the surgeon may repeat the method for a second time. Thus, second, the surgeon may disengage the endoscope from the light guide means which connect radiation source and endoscope. By holding the radiation exit region of the light guide means against the radiation sensor and conducting the method a second time, the surgeon can tell with certainty whether the endoscope one the one hand or the light guide means or the radiation source on the other hand cause the problem.

In a third step, the surgeon may disengage the light guide means from the radiation source and exchange the light guide means. After connecting the new light guide means and repeating the method for a third time, the surgeon can tell whether the light guide means caused the initial problem in case the feedback improved. In case the feedback did not improve, i.e. the registered luminous flux is not within an acceptable range, the surgeon at least knows that either the radiation source or the new light guide means causes the problem. Thus, the number of possible causes can at least be limited. Without the radiation sensor described herein, such a step-by-step-exchange of parts does not make any sense because the user does not have the possibility to test whether the exchange of a certain part solves the problem. Especially in surgery it is of course not possible to exchange a part, go on with the surgery and see if the result improved, since such an approach endangers the patient's health. Therefore, nowadays the surgeon would usually stop a minimal invasive surgery and lance or slice the respective body part of the patient to go on with an open surgery because there is no possibility to fix problems with the endoscopic system.

In brief, the parts of the system can be interchanged step by step until the defective part is identified.

The luminous flux of the radiation arriving at the sensor should be in a range above the detection threshold and below the saturation point where additional radiation, e.g. additional photons, cannot be registered by the saturated sensor. In case of saturation the intensity of the radiation source may be downregulated or other measures may be taken such as employing physical or digital filters or the like. Preferably, however, the embodiments described hereinafter are used in order to avoid saturation of the sensor.

Whereas the aforementioned method does not employ a device for transmission reduction, the present invention also comprises the following method for testing an optical system comprising a part to be tested:
- Placing a device for transmission reduction between the part to be tested and the radiation sensor
- Placing the radiation exit region of the part to be tested so that the radiation can be registered by the radiation sensor
- Activating the radiation source providing the part to be tested with radiation
- Registering at least a first luminous flux
- Providing a feedback on the luminous flux registered by the radiation sensor

The aforementioned steps may be conducted in the given order. Alternatively, the radiation source may be activated earlier as described above.

The device for transmission reduction may be a cap as described in detail above and the radiation exit region of the part to be tested may be covered with that cap. The radiation exit region may be placed by holding the cap against the radiation sensor. Preferably, the part to be tested is positioned such that the radiation passing through the cap falls onto the sensor.

The present invention also comprises a method wherein the following steps are conducted consecutively:
- Covering the radiation exit region of the part to be tested with a cap
- Holding the cap in position against a radiation sensor
- Registering a second luminous flux
- Activating the radiation source providing the part to be tested with radiation
- Registering a first luminous flux
- Providing a difference by subtracting the second registered luminous flux from the first registered luminous flux
- Providing a feedback on the difference

It is to be noted that the first luminous flux does not refer to the chronological order of steps but always refers to the luminous flux measured when the radiation source is activated. In contrast, the second luminous flux which is registered preferably but not mandatory refers to the luminous flux registered when the light source is not activated.

By using to the aforementioned method it is possible to register only the luminous flux originating from the radiation source since the radiation present in the environment of the sensor, e.g. the radiation originating from operating lamps, is removed by providing the difference.

The first luminous flux or the difference may be compared to previously stored values. Those previously stored values are either previously stored first luminous flux values or previously stored differences, respectively. Previously stored values may originate from various sources. Empirical or calculated values may be stored prior to using the method. Alternatively, those values may be obtained and stored during previous measurements collected during earlier performances oft he method.

Those previously stored values preferably serve as reference values.

As stated above, the previously stored values may be values of previous tests and the feedback may provide the currently registered luminous intensities or differences and at least one previously stored luminous flux or difference. Such a direct comparison with previous tests immediately shows the user a slow decline or deterioration which would otherwise not have been detected. The system may thus be sent to maintenance before problems occur during surgery.

According to an example of the present disclosure, the method and/or the system may ask the user to conduct the test method described above after a pre-defined time in which the radiation means was switched on or after the system has been used for a predefined number of times such as 5 or 10 times.

The present disclosure is directed at a method for testing an optical system which is highly useful in advance of and also during surgery. However, all the surgical steps, especially those steps applied in minimal-invasive surgery methods are conducted before the method steps of the present disclosure are conducted or without being functionally linked to the method steps of the present disclosure.

The method according to the present disclosure does not include a surgical step.

In examples of the method and the systems described herein an application on a mobile device or a remote control may be used for displaying data such as image data and/or feedback data and/or as means for recording input by the user. Hand-held mobile devices may be employed for this purpose. Mobile devices and/or remote control devices may be linked via known wireless methods to the systems described herein.

The configurations described above may be used in combination with a System comprising an optical instrument and method for operating that system described hereafter. In brief, hereafter an ambient radiation sensor is described which is configured to register ambient radiation conditions.

Radiation intensity and/or luminous flux caused by ambient radiation may be more than one hundred or one thousand times weaker than radiation intensity or luminous flux registered when the system described above is tested with the method described above. By using the device for transmission reduction described above, however, a common ambient radiation sensor may be employed for both functions. Therefore, according to a preferred example, only one sensor is used which allows for testing the optical system and operating that system with respect to registration of ambient radiation.

Hereafter, only the system comprising an optical instrument and method for operating that system which is focussed on ambient radiation is described.

A method for operating a system comprising an optical instrument for use in the field of medicine according to the present disclosure comprises the steps of registering ambient radiation of a pre-defined wavelength range and adjusting a radiation intensity of the radiation leaving the optical instrument and/or adjusting the brightness of the screen in response to the ambient radiation registered by an ambient radiation sensor. The system also comprises a radiation source and a screen or an eye piece for providing information to a user such as a surgeon. Usually this information mainly consists of the image registered by the optical instrument.

When referring to radiation or ambient radiation, especially the radiation registered by the sensor, preferably an intensity of that (ambient) radiation is meant herein.

Preferably, when a high intensity of ambient radiation is registered, the adjustment is done by increasing the radiation intensity of the radiation leaving the optical instrument and/or by increasing brightness of the screen. Correspondingly, when a low intensity of ambient radiation is registered, the adjustment is done by decreasing the radiation intensity of the radiation leaving the optical instrument and/or by decreasing brightness of the screen.

This method is suitably used with endoscopic or other systems comprising an optical instrument in the field of medicine, but may also applied in other areas, for example when operating borescopes.

The ambient radiation sensor may be any suitable sensor, for example a photodetector using the photoelectric effect.

In the context of the present disclosure, radiation may comprise visible light or infrared radiation. Also ultraviolet radiation may be comprised.

Visible light in the context of this invention is electromagnetic radiation within the wavelength range between 380 nm and 780 nm or between 400 nm and 700 nm.

Infrared radiation in the context of this invention is electromagnetic radiation within the wavelength range between 780 nm and 1 mm (1.000 µm) or between 700 nm and 1 mm (1.000 µm).

Near infrared radiation (NIR) in the context of this invention is electromagnetic radiation within the wavelength range between 780 nm and 1400 nm or between 700 nm and 1400 nm.

Systems comprising an eye piece such as an ocular lens are also encompassed by the present invention. However, preferably the present disclosure is directed at systems comprising a screen such as a flat panel display. Even more preferably the present invention is directed at endoscopic systems, especially at video endoscopes and video endoscopic systems.

The system preferably also comprises means for guiding the radiation of the radiation source to the area under investigation. Such means are present in known endoscopes.

The radiation intensity of the radiation leaving the optical instrument may be adjusted in a variety of ways. For example, the light source (or radiation source with respect to infrared radiation, for example) can be down-regulated or suitable automatically driven filters can be used.

The brightness of the screen can be adjusted in the way which is known from flat panel displays used with common personal computers and the like.

By adjusting the radiation intensity or the brightness of the screen as described above, the problems known from the state of the art can be overcome since the image brightness is optimized automatically and thus never too high or too low.

The advantages provided by the method described above are readily evident with respect to visible light applications, i.e. when the pre-defined wavelength range consists of at least a subrange of the visible light spectrum.

However, there are other advantages with respect to applications using infrared radiation, for example. When employing infrared radiation during surgery by using techniques such as NIR-fluorescence or thermography which are discussed in more detail below, the pre-defined wavelength range may consist of at least a subrange of the infrared range, e.g. the near infrared radiation (NIR) range. Thus, for example, the radiation intensity of the radiation leaving the optical instrument may be adjusted in response to the ambient infrared radiation. In this way interference of ambient infrared radiation is avoided and only remitted or fluorescent radiation which is to be investigated is detected by the optical instrument.

Preferably, said registering function and/or that said adjusting function can be disabled. Disabling one or both of the aforementioned functions is preferably possible at any given time, for example, before a surgery begins or even during the surgery.

There are rare situations in which a user, e.g. a surgeon, has to increase or decrease ambient radiation, especially artificial illumination such as ceiling lights or operating lights, to the maximal extent and accept aggravated image quality. In some of these situations the automatic adjustment of the radiation intensity and/or screen brightness described above is not advantageous. In order to allow for such deliberate minimization or maximization of ambient radiation without automatic adjustments conducted by the system, the disabling function described above may be provided.

Preferably, a feedback on the ambient radiation conditions is given to a user. The feedback may be provided by any part of the system.

The feedback may be provided immediately or shortly after starting or booting the system. Thus, when starting an endoscopic system prior to the surgery, for example, an immediate feedback on the ambient radiation conditions is given to the user. This may be advantageous for the user since the method and the system according to the present disclosure thus allow for testing the ambient radiation in the room by starting or booting the system. Thus, undesirable ambient radiation conditions may be changed before the surgery begins, since the system is usually booted some time prior to beginning the surgery.

However, ambient radiation conditions may change over time, e.g. because incident sunlight varies over time or simply because light sources of artificial illumination such as ceiling lights or operating lights are switched off or additional light sources are switched on during surgery. Therefore, preferably, the feedback function described herein is active all the time and checks for ambient light conditions as described below.

The feedback can be, for example, a visual feedback or an acoustic feedback. The visual feedback may be given on the flat panel display, e.g. by appropriate pictograms, numbers or the like.

The feedback may be generated by comparing the radiation intensity registered by the radiation sensor with radiation intensity values stored in the system or with radiation values registered during former surgeries or with radiation values pre-defined by the user or a combination of the aforementioned values. Suitably, at least a minimum and a maximum intensity of a preferred radiation intensity range are stored in the system.

After comparing the values the system may for example indicate the deviation of the registered radiation intensity from the values stored in the system. This may be done by giving the deviation in percent or by providing the radiation intensity in a suitable unit together with the registered information whether that radiation is in the optimal range or too low or too high. In short, for example relative or absolute radiation values may be given which may also be translated into qualitative statements which can readily be interpreted by the user. Additionally or alternatively the feedback may include instructions and guidance to the user by recommending increasing the ambient radiation in case the radiation intensity measured by the sensor is lower than a lower border of the preferred or optimal range. Accordingly, the instructions to reduce the ambient radiation may be given when the radiation intensity measured by the radiation sensor is above the upper border of the optimal or preferred intensity range.

As described above, the method according to the present disclosure comprises automatic adjustment of radiation intensity leaving the optical instrument and/or automatic adjustment of the brightness of the screen. However, in some situations the ambient radiation, especially ambient (visible) light may be extremely high or extremely low resulting in suboptimal overall light conditions. Therefore, upper and/or lower borders for the adjustment function may be included or implemented. Thus, the adjustment is only conducted within these borders and, for example, an excess of illumination in the operating room does not lead to the screen brightness to increase to values which even worsen the recognisability of the image.

Thus, also extremely unfavourable light conditions such as complete darkness do not result in the radiation intensity to drop to zero or near zero and/or to turn the screen dark. Such borders are of advantage in the rare occasions where such extremely unfavourable radiation conditions are accepted or even chosen deliberately for a specific reason. Such borders are also of advantage in occasions where such unfavourable conditions occur by chance or accident and respective adjustments would even worsen the situation, e.g. when turning the screen dark when the operating light turns dark.

On the one hand, as described above, there may be an upper and a lower border for the radiation intensity leaving the optical instrument and/or for the brightness of the screen. These borders prevent the system from conducting an adjustment which would even worsen the overall situation for the user, e.g. for the surgeon.

On the other hand, alternatively or additionally to the aforementioned borders, the feedback function described above is also advantageous in situations with highly undesirable ambient radiation conditions. Especially in combination, both functions are highly advantageous, since the adjustment is only conducted within acceptable borders without worsening the situation and at the same time the feedback is given instructing the user to improve the ambient radiation conditions. This guarantees optimal ambient radiation conditions all the time during surgery.

In short, there may be a range of ambient radiation where automatic adjustment is suitable while outside of that range adjustment even worsens the situation. This problem may be overcome by the method described in detail above, which comprises automatic adjustment within a pre-defined range of ambient radiation and which provides a feedback to the user when ambient radiation should be manually changed.

The aforementioned steps of course apply to visible light or visible radiation.

Moreover, the aforementioned steps also apply to infrared radiation, which is often used in surgery. In visible light applications the user, for example the surgeon, may at least partially judge ambient light intensity by himself and is supported by the adjustment function and/or by the feedback as described above. However, in infrared radiation applications the user or surgeon cannot judge ambient infrared radiation with his eyes. Thus, if the desired image cannot be obtained the user cannot even guess whether there is a technical problem or whether the system works properly and ambient infrared illumination is the problem.

Problems arise, for example, when remitted or fluorescent infrared radiation in the wavelength range of interest is present within ambient radiation. For example, especially near infrared radiation (NIR) constitutes a significant proportion of sunlight. Also many sources of artificial illumination generate a significant proportion of infrared radiation.

In the field of medicine, active and especially passive infrared thermography and NIR fluorescence are widely used. These processes are widely known.

When conducting minimal invasive surgery and using NIR fluorescence techniques, ambient near infrared radiation may be registered by the optical instrument because that radiation may in some way reach the area under investigation. Moreover, sometimes NIR techniques are used in open surgery, where, when present, ambient infrared radiation most certainly reaches the area under investigation.

In these situations the aforementioned steps provide a significant improvement to the surgeon. When, for example, the image sensor of the optical instrument such as a video endoscope is configured to register near infrared radiation, according to an embodiment of the present invention the system is configured so that the ambient radiation sensor automatically registers radiation in the near infrared wavelength range in order to detect interfering radiation. Thus, interference by ambient infrared radiation can be avoided.

Of course, this example may be transferred to any other wavelength range, i.e. the ambient radiation sensor may always be configured to detect ambient radiation in the wavelength range which is being investigated by the user with the help of the optical instrument.

When high ambient infrared radiation is detected, the radiation intensity of infrared radiation leaving the optical instrument may be adjusted, i.e. increased, accordingly.

Additionally, when ambient infrared radiation exceeds a pre-defined border, a feedback may be given to the user to reduce infrared ambient radiation which may be accomplished by reducing or even switching out artificial illumination, for example.

As described above, also with respect to infrared radiation there may be an optimal range within which, for example, automatic adjustment of the radiation intensity leaving the optical instrument is desirable; whereas outside of which providing a feedback to the user in order to change ambient infrared radiation conditions is more favourable.

Thus, as described in detail above, the pre-defined wavelength range may consist of infrared radiation.

Of course, alternative embodiments are encompassed by the present disclosure.

For example, the system may comprise two or more ambient radiation sensors. For example, a first ambient radiation sensor may register infrared radiation or NIR, while a second ambient radiation sensor may register visible light radiation. Such an embodiment comprising at least a first and a second ambient radiation sensor is advantageous when the system is used for both, infrared and visible light applications. Such an embodiment is especially advantageous when overlay-images consisting of the visible light image and the infrared image are to be provided.

The sensor may also be suitable for detecting both infrared and visible light radiation. The system may then split or divide the registered infrared radiation intensity and the registered visible light intensity, for example with digital filters or with prisms and physical filters or the like. Thus, the functions described above may be provided for both, ambient infrared radiation and ambient visible light by using only one ambient radiation sensor.

In general, at least a first and a second pre-defined wavelength range may be registered by at least one ambient radiation sensor.

Regardless of whether visible and infrared radiation is registered by one or two sensors, the system may comprise more than one radiation source. In case the system comprises an infrared radiation source and a visible light source, both radiation sources may be adjusted individually based on the registered radiation, i.e. the registered ambient visible light only leads to adjustment of the radiation intensity of the visible light source.

The method may comprise additional features.

For example, a test mode may be provided for the ambient radiation sensor. This test mode may at least comprise registering the radiation intensity of the pre-defined wavelength range without influence or manipulation of the user and registering the radiation intensity of the pre-defined wavelength when the user directs a part which emits radiation such as the distal end of the endoscope at the ambient radiation sensor. Thus, a fast and easy test can be conducted by guiding the user through the aforementioned steps and by comparing the registered intensity values with stored values reflecting acceptable intensity ranges for the chosen wavelength range in the at least two test situations, i.e. without manipulation by the user and with the user directing the endoscope at the sensor.

Although most functions and features were described with respect to endoscopes and endoscopic systems, these functions and features may also be included in methods for operating other systems and other optical instruments.

The present disclosure is directed at a method for operating a system as described above which is highly useful in surgery. However, all the surgical steps, especially those steps applied in minimal-invasive surgery methods are conducted before the method steps of the present invention are conducted or without being functionally linked to the method steps of the present invention. The method according to the present disclosure does not include a surgical step.

Besides the aforementioned process, the present disclosure is also directed at the system described below.

A system according to the present disclosure comprises an optical instrument for use in the field of medicine. The system comprises an ambient radiation sensor, a radiation source and a screen or an eye piece for providing information to a user. The ambient radiation sensor is configured to register ambient radiation of a pre-defined wavelength range. The system is configured to adjust a radiation intensity of the radiation source and/or a brightness of the screen in response to the registered ambient radiation.

The system may be an endoscopic system. Such systems comprise the endoscope, which may be connected to a control unit comprising the light source or radiation source. The radiation source or light source may also be a separate part which is not integrated in the control unit. Alternatively, the radiation source may also be integrated in the optical instrument, for example in the endoscope. The screen may be part of the control unit or it may be a separate part connected to that control unit. The ambient radiation sensor may be mounted in a housing of the control unit. The ambient radiation sensor may also be mounted elsewhere, for example on the endoscope itself at a place which is not introduced into the body of the patient and which is not covered by the hands of the surgeon during surgery.

Such a system is suitable for use in the medical field. However, such a system may also be used in a variety of other technical fields.

The system may be configured to give a feedback on the ambient radiation conditions. This feedback function is explained in more detail above with respect to the method according to the present invention.

The ambient radiation sensor may be configured to register ambient radiation of a pre-defined wavelength range of the infrared part of the spectrum.

The optical instrument may comprise an image sensor. Thus, in a preferred example, the system comprises an optical instrument which comprises an image sensor and the system additionally comprises an ambient radiation sensor.

The optical instrument may be an endoscope.

According to an embodiment of the present disclosure an endoscopic system is provided comprising an endoscope with an image sensor, a control unit and a radiation source. Such a system is known and commercially available. According to the present invention, the system is equipped with at least a first ambient radiation sensor which works as described in detail above. This ambient radiation sensor may be included in the housing of the control unit which may also comprise the radiation source.

The present disclosure also comprises the use of an ambient radiation sensor for operating a system comprising an optical instrument for use in the field of medicine comprising a radiation source and a screen or an eye piece. For example, a given endoscopic system may, according to the present disclosure, be equipped with an ambient radiation sensor which was described in detail above.

## Claims

1. **Endoscopic system** comprising an endoscope and a test system, the test system comprising a radiation sensor and the endoscope comprising a light guide means, wherein
the test system comprises a cap configured to cover a radiation exit region of the endoscope which is to be tested, and wherein the cap comprises a holding means and a lid portion
**characterised in that** the lid portion in turn comprises a semi-transparent material or is made of a semi-transparent material, said cap being thereby configured to ensure that in use the radiation sensor is not saturated by radiation exiting the radiation exit region of the endoscope.

2. **Method for testing an endoscopic system** according to claim 1, comprising the following steps:
- Covering a radiation exit region of the endoscope with the cap
- Placing the radiation exit region of the endoscope so that the radiation can be registered by the radiation sensor, wherein the radiation exit region is placed by holding the cap against the radiation sensor
- Activating a radiation source providing the endoscope with radiation
- Registering at least a first luminous flux
- Providing a feedback on the luminous flux registered by the radiation sensor

3. Method according to claim 2 wherein the following steps are conducted consecutively:
- Covering the radiation exit region of the part to be tested with a cap
- Holding the cap against a radiation sensor
- Registering a second luminous flux
- Activating the radiation source providing the part to be tested with radiation
- Registering a first luminous flux
- Providing a difference by subtracting the second registered luminous flux from the first registered luminous flux
- Providing a feedback on the difference

4. Method according to at least one of the claims 2 to 3, wherein the first luminous flux or the difference is compared to previously stored values.

5. Method according to at least one of the claims 2 to 4, **characterized in that** the previously stored values are values of previous tests and the feedback provides the currently registered luminous intensities or differences and at least one previously stored luminous flux or difference.

## Patentansprüche

1. Endoskopisches System, das ein Endoskop und ein Testsystem umfasst, wobei das Testsystem einen Strahlungssensor und das Endoskop ein Lichtleitmittel umfasst, wobei das Testsystem eine Kappe umfasst, die so konfiguriert ist, dass sie einen Strahlungsaustrittsbereich des zu testenden Endoskops abdeckt, und wobei die Kappe ein Haltemittel und einen Deckelteil umfasst
**dadurch gekennzeichnet,**
**dass** der Deckelteil seinerseits ein halbtransparentes Material umfasst, das aus einem halbtransparenten Material hergestellt ist, wobei die Kappe so konfiguriert ist, dass sie sicherstellt, dass der Strahlungssensor im Gebrauch nicht durch Strahlung gesättigt wird, die aus dem Strahlungsaustrittsbereich des Endoskops austritt.

2. Verfahren zum Testen eines endoskopischen Systems nach Anspruch 1, das folgende Schritte umfasst:
- Abdecken eines Strahlenaustrittsbereichs des Endoskops mit der Kappe
- Platzieren des Strahlungsaustrittsbereichs des Endoskops, so dass die Strahlung durch den Strahlungssensor registriert werden kann, wobei der Strahlungsaustrittsbereich durch Halten der Kappe gegen den Strahlungssensor platziert wird
- Aktivieren einer Strahlungsquelle, die das Endoskop mit Strahlung versorgt
- Erfassen mindestens eines ersten Lichtstroms
- Bereitstellen einer Rückmeldung über den vom Strahlungssensor registrierten Lichtstrom

3. Verfahren nach Anspruch 2, wobei die folgenden Schritte nacheinander durchgeführt werden:
- Abdecken des Strahlungsaustrittsbereichs des zu prüfenden Teils mit einer Kappe
- Halten der Kappe gegen einen Strahlungssensor
- Erfassen eines zweiten Lichtstroms
- Aktivieren der Strahlungsquelle, die das zu prüfende Teil mit Strahlung versorgt
- Erfassen eines ersten Lichtstroms
- Ermitteln der Differenz durch Subtraktion des zweiten registrierten Lichtstroms vom ersten registrierten Lichtstrom
- Bereitstellung einer Rückmeldung über die Differenz

4. Verfahren nach mindestens einem der Ansprüche 2 bis 3, wobei der erste Lichtstrom oder die Differenz mit zuvor gespeicherten Werten verglichen wird.

5. Verfahren nach mindestens einem der Ansprüche 2 bis 4, **dadurch gekennzeichnet, dass** die zuvor gespeicherten Werte, Werte früherer Tests sind und die Rückmeldung die aktuell registrierten Lichtstärken oder Differenzen und mindestens einen zuvor gespeicherten Lichtstrom oder eine Differenz liefert.

## Revendications

1. **Système d'endoscopie** comprenant un endoscope et un système de test, le système de test comprenant un capteur de rayonnement et l'endoscope comprenant un moyen de guidage de lumière, le système de test comprenant un capuchon conçu pour couvrir une région de sortie de rayonnement de l'endoscope qui doit être testé, et le capuchon comprenant un moyen de maintien et une partie de couvercle ;
**caractérisé en ce que** la partie de couvercle comprend à son tour un matériau semi-transparent ou est constitué d'un matériau semi-transparent, ledit capuchon étant ainsi conçu pour garantir qu'en utilisation le capteur de rayonnement n'est pas saturé par le rayonnement sortant de la région de sortie de rayonnement de l'endoscope.

2. **Procédé de test d'un système endoscopique** selon la revendication 1, comprenant les étapes suivantes :
- Couverture d'une région de sortie de rayonnement de l'endoscope avec le capuchon
- Placement de la région de sortie de rayonnement de l'endoscope de sorte que le rayonnement puisse être enregistré par le capteur de rayonnement, la région de sortie de rayonnement étant placée en maintenant le capuchon contre le capteur de rayonnement
- Activation d'une source de rayonnement alimentant l'endoscope en rayonnement
- Enregistrement d'au moins un premier flux lumineux
- Fourniture d'une rétroaction sur le flux lumineux enregistré par le capteur de rayonnement

3. Procédé selon la revendication 2, les étapes suivantes étant conduites consécutivement :
- Couverture de la zone de sortie de rayonnement de la pièce à tester avec un capuchon
- Maintien du capuchon contre un capteur de rayonnement
- Enregistrement d'un second flux lumineux
- Activation de la source de rayonnement alimentant la pièce à tester en rayonnement
- Enregistrement d'un premier flux lumineux
- Fourniture d'une différence en soustrayant le second flux lumineux enregistré du premier flux lumineux enregistré
- Réalisation d'une rétroaction sur la différence

4. Procédé selon au moins l'une des revendications 2 à 3, le premier flux lumineux ou la différence étant comparé à des valeurs précédemment enregistrées.

5. Procédé selon au moins l'une des revendications 2 à 4, **caractérisé en ce que** les valeurs précédemment mémorisées sont des valeurs de tests précédents et la rétroaction fournit les intensités ou différences lumineuses actuellement enregistrées et au moins un flux lumineux ou une différence lumineuse précédemment mémorisé(e).
